# EUROPÄISCHE PATENTSCHRIFT

(11) **EP 2 059 212 B1**
(45) Veröffentlichungstag und Bekanntmachung des Hinweises auf die Patenterteilung: **15.02.2012**
(21) Anmeldenummer: 07801941.1
(22) Anmeldetag: 28.08.2007
(51) Int. Cl.: A61J 1/14

(54) **MEHRZWECKKONNEKTOR ZUR ENTERALEN APPLIKATION**
MULTI-PURPOSE CONNECTOR FOR ENTERAL APPLICATION
CONNECTEUR POLYVALENT POUR ADMINISTRATION ENTÉRALE

(30) Priorität: 04.09.2006 DE 102006041414
(43) Veröffentlichungstag der Anmeldung: 20.05.2009
(73) Patentinhaber: Fresenius Kabi Deutschland GmbH, 61352 Bad Homburg (DE)
(72) Erfinder: BREUER-THAL, Barbara, 65510 Idstein (DE); OSTER, Marcel, 41065 Mönchengladbach (DE)
(74) Vertreter: Sundermann, Corinna
(86) Internationale Anmeldenummer: PCT/EP2007/007519
(87) Internationale Veröffentlichungsnummer: WO 2008/028582

(56) Entgegenhaltungen:
- EP-A- 0 355 795
- EP-A- 0 711 538
- US-A- 3 019 932
- US-A- 4 969 565
- US-B1- 6 875 204

## Beschreibung

Die Erfindung betrifft einen Mehrzweckkonnektor zum Anschluß von enteralen Überleitsystemen an Depotbehältnisse, insbesondere an Nahrungs- und Bilanzierungsflüssigkeitsbehältnisse, mit verschiedenartig gestalteten Öffnungen nach dem Oberbegriff des Anspruchs 1. Der Konnektor kann in der enteralen Ernährung sowohl bei pumpenassistierten als auch bei schwerkraftgesteuerten Systemen zum Einsatz kommen. Die Erfindung bezieht sich auch auf ein Set mit mindestens einem derartigen Mehrzweckkonnektor. Darüber hinaus betrifft die Erfindung die Verwendung des Mehrzweckkonnektors oder eines Sets mit mindestens einem derartigen Mehrzweckkonnektor.

Ist ein Patient aufgrund einer Erkrankung nicht mehr in der Lage, Nahrung aufzunehmen oder zu schlucken, können ihm die wichtigsten Nährstoffe in flüssiger Form über eine Emährungssonde zugeführt werden.

Die Ernährungssonde wird entweder vorübergehend durch die Nase in den Magen oder Dünndarm geführt oder sie wird dauerhaft über eine kleine Einstichstelle durch die Bauchdecke im Magen oder Dünndarm plaziert. Ein Überleitsystem verbindet die Emährungssonde mit dem Nahrungs- und Bilanzierungsflüssigkeitsbehältnis.

Das Überleitsystem besteht aus einem Schlauch mit oder ohne Tropfkammer, einem distalen Konnektor zur Verbindung mit dem enteralen Depotbehältnis und einem proximalen Anschluß an die Ernährungssonde.

Die enterale Nährstofflösung oder Bilanzierungsflüssigkeit wird in Containern, Flaschen oder Beuteln aufbewahrt, deren Öffnungen zwar normiert oder mindestens standardisiert sind, jedoch unterschiedlich gestaltet sein können. Es sind u. a. verbreitet: Weithalsbehältnisse, beispielsweise Container oder Flaschen aus Glas oder Kunststoff mit Weithalsgewinden und zusätzlicher Aluminium- oder Kunststoffschutzfolie, ferner Enghalsbehältnisse, wie zum Beispiel Kronenkorkenflaschen mit Mundstück gemäß DIN 6094 aus Glas oder Hartkunststoff, und flexible Behältnisse, wie z. B. vorgefüllte Kunststoffbeutel mit Einstichport.

Die unterschiedlich geformten Depotbehältnisöffnungen erfordern auch unterschiedliche, jeweils angepaßte Konnektoren zum Anschluß an das Überleitsystem. Die Bereitstellung unterschiedlicher, auf den jeweiligen Depotbehältnistyp angepasster Konnektoren erhöht jedoch die Kosten und führt außerdem zu einem gesteigerten Handlingsaufwand, wie z. B. der Notwendigkeit des umständlichen Aussuchens eines geeigneten Konnektors, der Vorratshaltung und des Konnektorwechsels während der Applikation.

Aus der EP 0 355 795 ist ein Adapter zum Anschluß von enteralen Überleitsystemen bekannt. Der Adapter ist aus Kunststoff gefertigt und weist zwei koaxial zueinander angeordnete Kappen mit verschiedenen Öffnungsdurchmessem auf. Es fehlt jedoch die Integration eines Dorns, so dass zur Konnektion an die in der enteralen Applikation zu mehr als 50% vertretenen, als Beutel oder Kunststoffbehältnisse mit Portanschluß ausgeführten Primärpackungsbehältnisse ein zusätzlicher Konnektor mit Überleitsystem notwendig ist. Nachteilig ist ferner die Positionierung des Belüftungsventils an der Innenseite des Konnektors bzw. im Inneren des Nahrungs- und Bilanzierungsflüssigkeitsbehältnisses, da es zu Verstopfungen und nachfolgendem Föderflussstopp kommen kann.

Aus der US 6,875,204 ist ein Universalkonnektor bekannt, an den Behältnisse mit drei verschiedenen Öffnungsdurchmessern angeschlossen werden können, wobei ein Dorn eine der Anschlußmöglichkeiten darstellt. Bei diesem bekannten Konnektor fehlt jedoch eine Belüftungsöffnung, so dass sich dieser nicht für den Einsatz an starren Behältnissen eignet. Des weiteren kann der Konnektor nicht direkt mit einem Überleitsystem verbunden, sondern lediglich in ein Weithalsflaschensystem eingedreht werden.

Aus der EP 0 711 538 ist ein Universalflaschenverschluß mit zwei unterschiedlich großen, koaxial angeordneten Kappen bekannt. Die Kappen bestehen aus unterschiedlichem Material. Nachteilig ist hier zum einen der fehlende Dorn und zum anderen das für die Belüftung integrierte Steigrohr. Letzteres bedingt, dass nach der Applikation noch Testflüssigkeit im Behältnis verbleibt.

Der Erfindung liegt daher die Aufgabe zugrunde, einen Mehrzweckkonnektor zum Anschluß von enteralen Überleitsystemen an Depotbehältnisse mit verschiedenartig gestalteten Öffnungen zu schaffen, der vielfältige Einsatzmöglichkeiten aufweist.

Diese Aufgabe wird durch einen Mehrzweckkonnektor mit den Merkmalen des Anspruchs 1 gelöst.

Der erfindungsgemäße Mehrzweckkonnektor besitzt drei verschiedene distale und zwei verschiedene proximale Anschlüsse. Sämtliche Anschlüsse liegen konzentrisch ineinander und koaxial zueinander. Auf der dem Patienten abgewandten, d. h. distalen Seite befinden sich ein äußerer, ein mittlerer und ein innerer Anschluß, die jeweils unterschiedlich gestaltet sind. Auf diese Weise können Weit- und Enghalsbehältnisse aus Glas oder Kunststoff oder auch flexible Depotbehältnisse mit Einstichport konnektiert werden. Aufgrund des durch die Vereinigung verschiedener Depotbehältnisvarianten gestrafften Produktportfolio und der dadurch entstehenden Reduzierung von Material- und Montageaufwand ergeben sich die Vorteile einer sehr kostengünstigen Herstellung. Weitere Vorteile des erfindungsgemäßen Konnektors für den Anwender liegen in der vereinfachten Handhabung und der weniger aufwändigen Vorratshaltung. Die verschiedenen Depotbehältnisse bzw. Nahrungsbehältnisse können beispielsweise auch alternierend eingesetzt werden, ohne dass gleichzeitig der Konnektor gewechselt werden muß.

Auf der gegenüberliegenden, dem Patienten zugewandten, d. h. proximalen Seite befinden sich zwei weitere, ebenfalls konzentrisch ineinander und koaxial zueinander liegende Anschlüsse zur direkten Montage mit einer Tropfkammer oder einem Schlauch.

Vorteilhafte Ausführungsformen sind Gegenstand der Unteransprüche.

Der Mehrzweckkonnektor ist ein zweistückiges, aus Kunststoff gefertigtes Spritzgußteil und damit kostengünstig in der Herstellung. Ein Teilstück besteht dabei aus dem äußeren, dem mittleren und einem Teil des inneren distalen Anschlusses und den beiden proximalen Anschlüssen. Beide Teilstücke werden bei der Fertigung ineinandergesteckt und verrasten dabei. Vorteilhaft bei der zweistückigen Herstellung ist die Möglichkeit, verschiedene Materialtypen oder Materialhärtegrade verwenden zu können. Es ist jedoch auch möglich, den Mehrzweckkonnektor einstükkig herzustellen.

In einer Ausgestaltung der Erfindung ist der äußere distale Anschluss mit einem Schraubgewinde versehen, der mittlere distale Anschluss als Schnappverbindung und der innere distale Anschluß als Führung mit Gewinde und einem zentralen Dorn ausgebildet. Der zentrale Dorn besitzt seitliche Materialaussparungen, die die Bildung von Restflüssigkeit verhindert.

An den äußeren Anschluß können Weithalsbehältnisse, beispielsweise Flaschen mit einem Weithalsgewinde, und an den mittleren Anschluß Enghalsbehältnisse, wie zum Beispiele Kronenkorkenflaschen, konnektiert werden. Der innere Anschluß ist geeignet für flexible Behältnisse, beispielsweise Kunststoffbeutel mit einem Einstichport. Durch die Verwendung des Doms in Kombination mit einem Konnektor ist eine Verwechslung mit intravenösen Dornen ausgeschlossen. Zudem wird der Konnektor nicht, wie bisher üblich, auf ein Dornsystem gedreht, sondern vorteilhafterweise direkt mit der Tropfkammer oder dem Überleitsystemschlauch verbunden, was zusätzliche Material- und Montagekosten einspart.

Der erfindungsgemäße Mehrzweckkonnektor besitzt eine Entlüftung. Zu diesem Zweck ist, in den mittleren distalen Anschluß mündend, ein Belüftungskanal vorgesehen, dessen innere Öffnung bündig mit dem Boden des mittleren Anschlusses abschließt. Eine Bildung von Restflüssigkeit, wie es bei dem aus der EP 0 711 538 bekannten Universalflaschenverschluss aufgrund des Vorhandenseins eines über den Boden des Konnektors herausragenden Steigrohres geschieht, wird dadurch vermieden.

Im Außenbereich des Konnektors befindet sich die äußere Belüftungskanalöffnung, in die eine Baugruppe mit einem bakteriendichten Filter und einem Belüftungsventil eingesetzt werden kann. Diese Positionierung im Außenbereich verhindert ein Verstopfen und damit eine Unterbrechung der Nahrungsförderung. Die Nahrungsförderung wird auch durch das Design begünstigt, das nur geringere Kontaktflächen zwischen Applikationsmedium und Konnektor zuläßt.

Die Erfindung betrifft nach Anspruch 16 weiter ein Set zur enterealen Applikation, das aus einem Überleitsystem und dem erfindungsgemäßen Mehzweckkonnektor besteht, und den Anschluß von Depotbehältnissen mit verschiedenartig gestalteten Öffnungen an die Emährungssonde eines Patienten ermöglicht. Vorteilhafte Ausführungsformen sind Gegenstand der von Anspruch 16 abhängigen Ansprüche.

Darüber hinaus ist die Verwendung eines Mehrzweckkonnektors oder eines Sets mit mindestens einem derartigen Mehrzweckkonnektor nach Patentanspruch 17 Gegenstand der Erfindung.

In einer Weiterbildung der Erfindung kann der Konnektor aus unterschiedlichen, dem Fachmann bekannten, geeigneten Materialien bestehen. In einer besondern Ausführungsform ist das erste Teilstück des Konnektors transparent. Dies macht den Dorn sichtbar und erleichtert dem Anwender den Einstichprozeß.

Der Mehrzweckkonnektor kann mit verschiedenartigen, dem Fachmann geläufigen Verbindungsmöglichkeiten, wie beispielsweise Schraub,- Schnapp- oder Steckverbindungen versehen sein.

Das erfindungsgemäße Set zum Anschluß von Nahrungs- und Bilanzierungsflüssigkeitsbehältnissen mit verschiedenartig gestalteten Öffnungen an eine Ernährungssonde besteht aus einem Überleitsystem und dem erfindungsgemäßen Mehrzweckkonnektor. Das Set kann eine Tropfkammer enthalten, die Verbindung zwischen Überleitsystem und einem der beiden proximalen Anschlüsse des Mehrzweckkonnektors kann aber auch lediglich über einen Schlauch hergestellt werden.

Weitere Einzelheiten der Erfindung werden anhand eines in den Zeichnungen dargestellten Ausführungsbeispiels näher beschrieben. Es zeigen:
**Fig. 1** einen eindimensionalen Längsschnitt durch eine Ausführungsform des erfindungsgemäßen Mehrzweckkonnektors,
**Fig. 2** einen dreidimensionalen Längsschnitt durch eine Ausführungsform des erfindungsgemäßen Mehrzweckkonnektors,
**Fig. 3a-c** verschiedene Ansichten des in den Fig. 1 und 2 gezeigten erfindungsgemäßen Mehrzweckkonnektors,
**Fig. 4a-g** verschiedene Ansichten des ersten Teilstücks des erfindungsgemäßen Mehrzweckkonnektors,
**Fig. 5a-f** verschiedene Ansichten des zweiten Teilstücks des erfindungsgemäßen Mehrzweckkonnektors und
**Fig. 6** einen eindimensionalen Längsschnitt durch eine alternative, einstückige Ausführungsform eines erfindungsgemäßen Mehrzweckkonnektors.

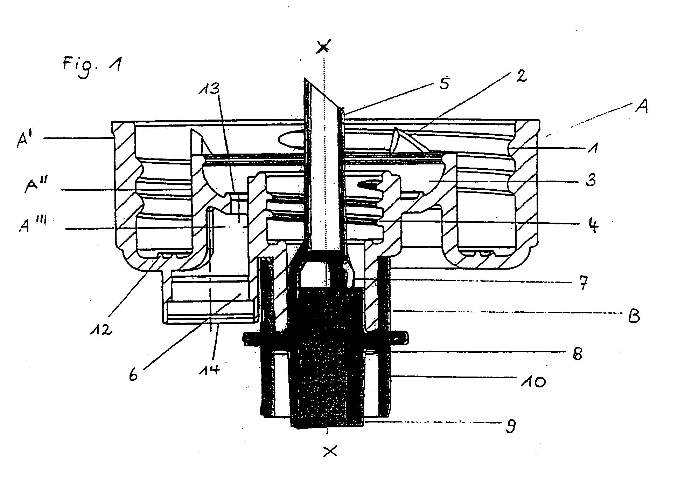

Der in der **Fig. 1** dargestellte Mehrzweckkonnektor besteht aus einem ersten Teilstück A und einem zweiten Teilstück B. Das erste Teilstück A beinhaltet einen äußeren Anschluß A' mit einem Innengewinde 1 zur Konnektierung von Weithalsflaschen aus Glas oder Kunststoff und Dichtrippen 12, einen mittleren Anschluß A" mit einer Schnappverbindung 3 zur Konnektierung von Enghalsflaschen und einen inneren Anschluß A'" als Führung mit einem Innengewinde 4 zur Konnektierung von Beuteln mit Einstichports. Auf dem Rand des mittleren Anschlusses befindet sich ein Schneidezahn 2 zum Öffnen von Deckelfolien bei Weithalscontainern. Das zweite Teilstück B besitzt als distalen Anschluß einen zentralen Dorn 5 zum Einstechen in Ports. Seitliche Materialaussparungen 7 an der Basis des Dorns vermeiden die Bildung von Restflüssigkeit. Die proximale Seite des Teilstücks B ist rohrförmig und dient als Tropfenbilder 8 bei der schwerkraftbetriebenen enteralen Ernährung. Am Ende des Tropfenbilders 8 befindet sich eine Schlauchanklebungsstelle 9 für die pumpenassistierte enterale Ernährung. Die Montage einer Tropfkammer erfolgt über den Vorsprung 10. Alle Anschlüsse sind koaxial in Bezug auf eine gemeinsame Achse X-X angeordnet.

Ausgehend von dem mittleren distalen Anschluß ist ein Belüftungskanal 6 in den Mehrzweckkonnektor integriert. In diesen kann eine Baugruppe, bestehend aus einem außenliegenden Ventil und einem bakteriendichten Filter eingesetzt werden. Die innere Belüftungskanalöffnung 13 befindet sich im mittleren distalen Anschluß und schließt weitgehend bündig mit dessen Boden ab. Die äußere Belüftungskanalöffnung 14 ist vertikal gerichtet.

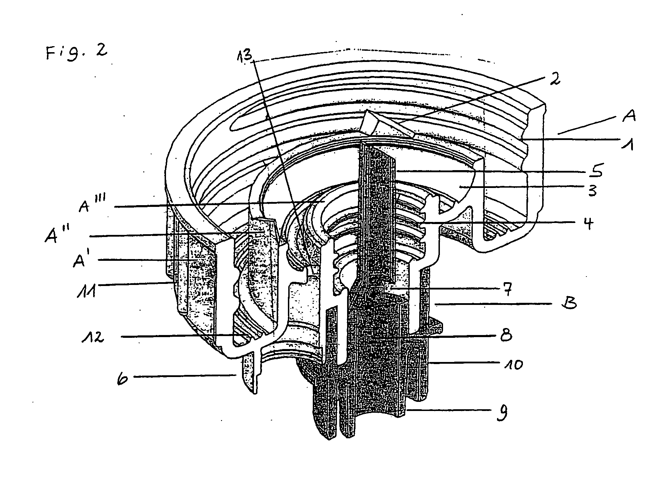

Die außen am Mehrzweckkonnektor angebrachten Grifflaschen 11 sind auf **Fig. 2** zu sehen.

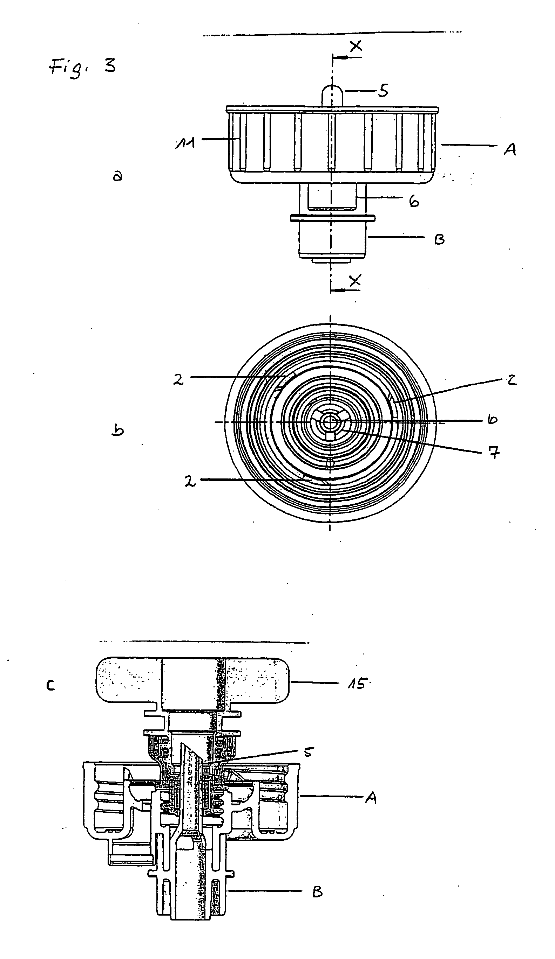

**Fig. 3** zeigt den erfindungsgemäßen Mehrzweckkonnektor in verschiedenen Ansichten, wobei 3a eine Außenansicht, 3b einen Querschnitt und 3c einen Längsschnitt mit konnektiertem Port 15 darstellen.

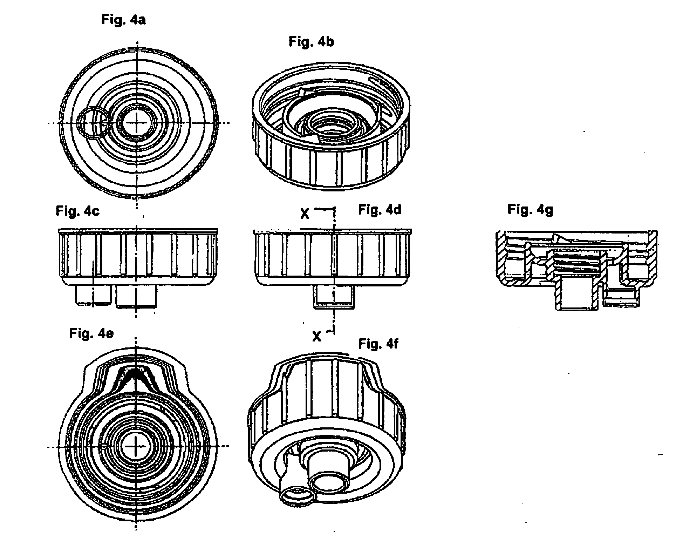

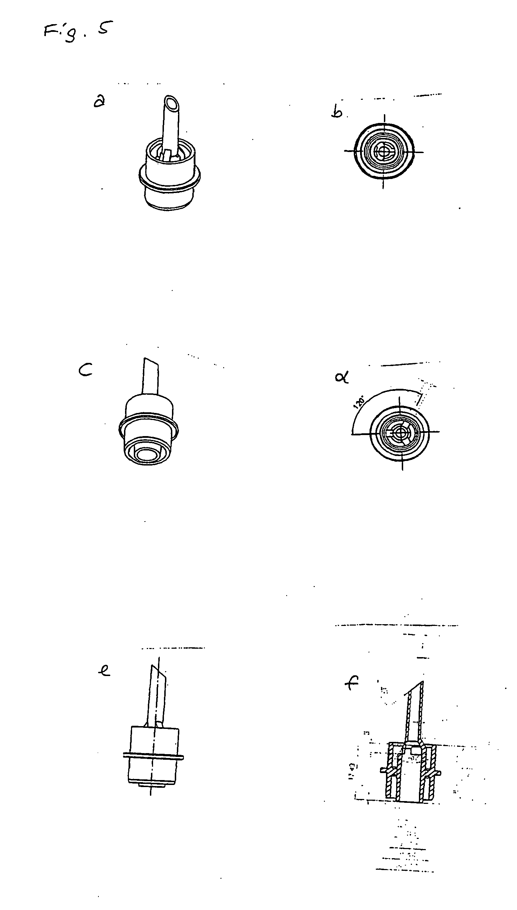

In **Fig. 4a-g** werden verschiedene Ansichten des ersten Teilstücks des erfindungsgemäßen Mehrzweckkonnektors dargestellt, in **Fig. 5a-f** verschiedne Ansichten des zweiten Teilstücks.

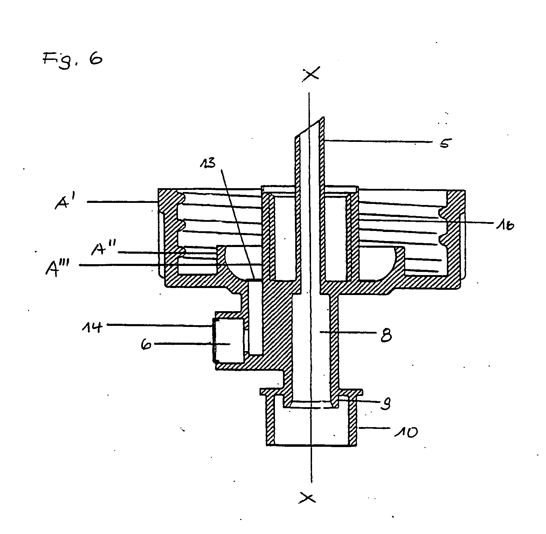

**Fig. 6** zeigt eine Alternative des Mehrzweckkonnektors als einstückige Ausführungsform. Der Belüftungskanal 6 befindet sich ebenfalls im Außenbereich des Konnektors.

Die innere Belüftungsöffnung 13 befindet sich nach wie vor im mittleren distalen Anschluß und schließt weitgehend bündig mit dessen Boden ab, die äußere Belüftungskanalöffnung 14 ist jedoch horizontal gerichtet.

Nach der Konnektierung eines Depotbehältnisses durch Aufschrauben oder Aufsetzen auf einen der Anschlüsse fließt das Applikationsmedium durch den Mehrzweckkonnektor entlang der Linie X-X über eine Tropfkammer oder einen Schlauch in das Überleitsystem. Die Belüftungsöffnung 6 gewährleistet einen ungestörten Ausfluß.

## Patentansprüche

1. Mehrzweckkonnektor mit drei verschiedenen, konzentrisch ineinander und koaxial zueinander liegenden distalen Anschlussen (A', A", A"') zur Konnektion von enteralen Überleitsystemen an Depotbehältnisse mit verschiedenartig gestalteten Öffnungen, wobei sich auf der gegenüberliegenden proximalen Seite der Anschlüsse für die Depotbehältnisse, insbesondere Nahrungs- und Bilanzierungsflüssigkeitsbehältnisse, zwei weitere Anschlüsse (9,10) befinden, **dadurch gekennzeichnet, dass** ein Belüftungskanal (6) vorgesehen ist, dessen innere Öffnung bündig mit dem Boden des mittleren distalen Anschlusses abschließt.

2. Mehrzweckkonnektor nach Anspruch 1, **dadurch gekennzeichnet, dass** an einen der beiden proximalen Anschlüsse ein Schlauch und an den anderen eine Tropfkammer konnektiert werden kann.

3. Mehrzweckkonnektor nach Anspruch 1 und 2, **dadurch gekennzeichnet, dass** er zweistückig ist.

4. Mehrzweckkonnektor nach Anspruch 3, **dadurch gekennzeichnet, dass** das erste Teilstück aus dem äußeren und dem mittleren distalen Anschluss und einem Teil des inneren distalen Anschlusses und das zweite Teilstück aus dem andern Teil des inneren distalen Anschlusses und den beiden proximalen Anschlüssen besteht.

5. Mehrzweckkonnektor nach Anspruch 3 oder 4, **dadurch gekennzeichnet, dass** das der Belüftungskanal im ersten Teilstück vorgesehen ist.

6. Mehrzweckkonnektor nach Anspruch 5, **dadurch gekennzeichnet, dass** in den Belüftungskanal ein bakteriendichter Filter und ein Ventil als außenliegende Baugruppe einsetzbar sind.

7. Mehrzweckkonnektor nach Anspruch 5 und 6, **dadurch gekennzeichnet, dass** die innere Belüftungskanalöffnung im mittleren distalen Anschluß angeordnet ist.

8. Mehrzweckkonnektor nach einem oder mehreren der vorhergehenden Ansprüche, **dadurch gekennzeichnet, dass** das erste Teilstück transparent ist.

9. Mehrzweckkonnektor nach einem oder mehreren der vorhergehenden Ansprüche, **dadurch gekennzeichnet, dass** der äußere distale Anschluß mit einem Schraubgewinde versehen ist.

10. Mehrzweckkonnektor nach einem oder mehreren der vorhergehenden Ansprüche, **dadurch gekennzeichnet, dass** der mittlere distale Anschluß als Schnappverbindung ausgebildet ist.

11. Mehrzweckkonnektor nach einem oder mehreren der vorhergehenden Ansprüche, **dadurch gekennzeichnet, dass** der innere distale Anschluß aus einer Führung mit einem Gewinde und einem zentralen Dorn besteht.

12. Mehrzweckkonnektor nach einem oder mehreren der vorhergehenden Ansprüche, **dadurch gekennzeichnet, dass** der zentrale Dorn seitliche Materialaussparungen zum besseren Ablauf von Flüssigkeit besitzt.

13. Mehrzweckkonnektor nach Anspruch 9, **dadurch gekennzeichnet, dass** an den äußeren Anschluß Weithalsbehältnisse, beispielsweise Flaschen mit einem außenliegenden Weithalsgewinde, konnektierbar sind.

14. Mehrzweckkonnektor nach Anspruch 10, **dadurch gekennzeichnet, dass** an den mittleren Anschluß Enghalsbehältnisse, wie zum Beispiel Kronenkorkenflaschen, konnektierbar sind.

15. Mehrzweckkonnektor nach Anspruch 11, **dadurch gekennzeichnet, dass** an den inneren Anschluß flexible Behältnisse, beispielsweise Kunststoffbeutel mit einem Einstichport, konnektierbar sind.

16. Set zum Anschluß von Nahrungs- und Bilanzierungsflüssigkeitsbehältnissen an eine Ernährungssonde mit mindestens einem Mehrzweckkonnektor nach einem der Ansprüche 1 bis 15.

17. Verwendung eines Mehrzweckkonnektors oder eines Sets nach einem oder mehreren der vorhergehenden Ansprüche zum Anschluß von enteralen Überleitsystemen an Nahrungs- und Bilanzierungsflüssigkeitsbehältnisse mit verschiedenartig gestalteten Öffnungen.

## Claims

1. Multi-purpose connector with three different distal attachments (A', A", A"') lying concentrically in one another and coaxially with respect to one another for the connection of enteral transfer systems to storage containers having differently configured openings, wherein two further attachments (9, 10) are located on the opposite, proximal side of the attachments for the storage containers, in particular containers holding nutrients and balance liquid, **characterized in that** a ventilation channel (6) is provided, of which the inner opening ends flush with the bottom of the middle distal attachment.

2. Multi-purpose connector according to Claim 1, **characterized in that** a hose can be connected to one of the two proximal attachments, and a drip chamber can be connected to the other proximal attachment.

3. Multi-purpose connector according to Claims 1 and 2, **characterized in that** it is in two pieces.

4. Multi-purpose connector according to Claim 3, **characterized in that** the first piece is composed of the outer distal attachment and middle distal attachment and of a part of the inner distal attachment, and the second piece is composed of the other part of the inner distal attachment and the two proximal attachments.

5. Multi-purpose connector according to Claim 3 or 4, **characterized in that** the ventilation channel is provided in the first piece.

6. Multi-purpose connector according to Claim 5, **characterized in that** a bacteria-proof filter and a valve can be inserted as an outer assembly into the ventilation channel.

7. Multi-purpose connector according to Claims 5 and 6, **characterized in that** the inner opening of the ventilation channel is arranged in the middle distal attachment.

8. Multi-purpose connector according to one or more of the preceding claims, **characterized in that** the first piece is transparent.

9. Multi-purpose connector according to one or more of the preceding claims, **characterized in that** the outer distal attachment is provided with a screw thread.

10. Multi-purpose connector according to one or more of the preceding claims, **characterized in that** the middle distal attachment is designed as a snap-fit connection.

11. Multi-purpose connector according to one or more of the preceding claims, **characterized in that** the inner distal attachment is composed of a guide with a thread and a central spike.

12. Multi-purpose connector according to one or more of the preceding claims, **characterized in that** the central spike has lateral material cut-outs for better drainage of liquid.

13. Multi-purpose connector according to Claim 9, **characterized in that** wide-mouth containers, for example bottles with an outward wide-mouth thread, can be connected to the outer attachment.

14. Multi-purpose connector according to Claim 10, **characterized in that** narrow-mouth containers, for example crown cap bottles, can be connected to the middle attachment.

15. Multi-purpose connector according to Claim 11, **characterized in that** flexible containers, for example plastic bags with a puncture port, can be connected to the inner attachment.

16. Set for attaching containers holding nutrients and balance liquid to a feed tube, having at least one multi-purpose connector according to one of Claims 1 to 15.

17. Use of a multi-purpose connector or of a set according to one or more of the preceding claims, for attaching enteral transfer systems to containers that hold nutrients and balance liquid and have differently configured openings.

## Revendications

1. Connecteur polyvalent avec trois raccords distaux différents (A', A", A"') disposés de façon concentrique les uns dans les autres et coaxialement les uns par rapport aux autres, pour la connexion à des systèmes de transfert entéral à des récipients de dépôt avec des ouvertures de formes différentes, dans lequel sont présents deux autres raccords (9, 10) sur le côté proximal opposé des raccords pour les récipients de dépôt, en particulier des récipients de liquide d'alimentation et d'équilibrage, **caractérisé en ce qu'**il est prévu un canal d'aération (6), dont l'ouverture intérieure se termine à fleur du fond du raccord distal médian.

2. Connecteur polyvalent selon la revendication 1, **caractérisé en ce qu'**un tuyau souple peut être connecté à un des deux raccords proximaux et une chambre à compte-gouttes peut être connectée à l'autre.

3. Connecteur polyvalent selon la revendication 1 et 2, **caractérisé en ce qu'**il est en deux parties.

4. Connecteur polyvalent selon la revendication 3, **caractérisé en ce que** la première partie est composée des raccords distaux extérieur et médian et d'une partie du raccord distal intérieur et la deuxième partie est composée de l'autre partie du raccord distal intérieur et des deux raccords proximaux.

5. Connecteur polyvalent selon la revendication 3 ou 4, **caractérisé en ce que** le canal d'aération est prévu dans la première partie.

6. Connecteur polyvalent selon la revendication 5, **caractérisé en ce qu'**un filtre antibactérien et une soupape peuvent être insérés sous forme de module extérieur dans le canal d'aération.

7. Connecteur polyvalent selon la revendication 5 et 6, **caractérisé en ce que** l'ouverture intérieure du canal d'aération est disposée dans le raccord distal médian.

8. Connecteur polyvalent selon une ou plusieurs des revendications précédentes, **caractérisé en ce que** la première partie est transparente.

9. Connecteur polyvalent selon une ou plusieurs des revendications précédentes, **caractérisé en ce que** le raccord distal extérieur est muni d'un filet de vis.

10. Connecteur polyvalent selon une ou plusieurs des revendications précédentes, **caractérisé en ce que** le raccord distal médian est réalisé sous la forme d'un assemblage à déclic.

11. Connecteur polyvalent selon une ou plusieurs des revendications précédentes, **caractérisé en ce que** le raccord distal intérieur est composé d'un guide avec un filet et un mandrin central.

12. Connecteur polyvalent selon une ou plusieurs des revendications précédentes, **caractérisé en ce que** le mandrin central présente des évidements de matière latéraux pour améliorer l'écoulement du liquide.

13. Connecteur polyvalent selon la revendication 9, **caractérisé en ce que** des récipients à col large, par exemple des flacons avec un large col extérieur fileté, peuvent être connectés au raccord extérieur.

14. Connecteur polyvalent selon la revendication 10, **caractérisé en ce que** des récipients à col étroit, comme par exemple des flacons à capsule de liège, peuvent être connectés au raccord médian.

15. Connecteur polyvalent selon la revendication 11, **caractérisé en ce que** des récipients souples, par exemple des poches en matière synthétique avec orifice à enfoncer, peuvent être connectés au raccord intérieur.

16. Ensemble destiné au raccordement de récipients de liquide d'alimentation et d'équilibrage à une sonde d'alimentation avec au moins un connecteur polyvalent selon l'une quelconque des revendications 1 à 15.

17. Utilisation d'un connecteur polyvalent ou d'un ensemble selon une ou plusieurs des revendications précédentes pour le raccordement de systèmes de transfert entéral à des récipients de liquide d'alimentation et d'équilibrage avec des ouvertures de formes différentes.
